# EUROPÄISCHE PATENTSCHRIFT

(11) **EP 3 364 943 B1**
(45) Veröffentlichungstag und Bekanntmachung des Hinweises auf die Patenterteilung: **23.06.2021**
(21) Anmeldenummer: 16781133.0
(22) Anmeldetag: 13.10.2016
(51) Int. Cl.: A61Q 13/00, A61K 8/04, A61K 8/81, A61K 9/00, A61K 9/08, A61K 9/12, A61K 47/08, A61K 47/10

(54) **FORMULIERUNGEN MIT KONTROLLIERTER FREISETZUNG VON GERUCHSSTOFFEN ZUR DERMALEN ANWENDUNG**
FORMULATIONS WITH A CONTROLLED RELEASE OF PERFUMES FOR DERMAL APPLICATION
FORMULATIONS À LIBÉRATION CONTRÔLÉE DE SUBSTANCES ODORANTES POUR APPLICATION DERMIQUE

(30) Priorität: 23.10.2015 EP 15191157
(43) Veröffentlichungstag der Anmeldung: 29.08.2018
(73) Patentinhaber: BASF SE, 67056 Ludwigshafen am Rhein (DE)
(72) Erfinder: KOLTER, Karl, 67117 Limburgerhof (DE); PELZER, Ralf, 37699 Fuerstenberg (DE); BENDEL, Marion, 67067 Ludwigshafen (DE); KARL, Matthias, 68163 Mannheim (DE)
(74) Vertreter: BASF IP Association
(86) Internationale Anmeldenummer: PCT/EP2016/074595
(87) Internationale Veröffentlichungsnummer: WO 2017/067843

(56) Entgegenhaltungen:
- EP-A2- 0 384 034
- US-B1- 6 172 037

## Beschreibung

Die vorliegende Erfindung betrifft Formulierungen mit kontrollierter Freisetzung von Geruchsstoffen zur dermalen Anwendung, wobei die Geruchsstoffe in einer Matrix auf Basis von einem wasserunlöslichen Polyvinylacetat mit einem mittleren Molekulargewicht Mw von 200000 bis 700000 Dalton und einem wasserlöslichen Homopolymer des N-Vinylpyrrolidons mit einem K-Wert von 30 eingebettet vorliegen.

Üblicherweise werden Geruchsstoffe als alkoholische Lösung auf die Haut gebracht. Aufgrund ihres relativ niedrigen Siedepunktes verflüchtigen sie sich und erzeugen auf diese Weise beim Einatmen einen bestimmten Geruch. Die Verflüchtigung wird noch verstärkt durch die Hauttemperatur von ca. 32°C und die relativ große Oberfläche der Haut und der darauf befindlichen Haare. Aufgrund dieser Gegebenheiten sind die Geruchsstoffe häufig schon nach kurzer Zeit nicht mehr intensiv wahrzunehmen. Hinzu kommt, dass die Geruchsstoffe in der Regel als Mischung von zahlreichen Komponenten z. B. in Form eines Parfüms eingesetzt werden, die alle einen unterschiedlichen Siedepunkt und somit eine unterschiedliche Flüchtigkeit aufweisen. Somit verändert sich der Geruch des Parfüms im Laufe der Anwendung, weil die leichter flüchtigen zuerst verdampfen.

Nach dem Stand der Technik wurde die verlangsamte Freisetzung von Geruchsstoffen vor allem durch Mikroverkapselung erreicht. Hierzu werden in der Regel intermediär Emulsionen hergestellt, die z. Teil anschließend getrocknet werden, bevor sie zur endgültigen Formulierung verarbeitet werden. Solche Formulierungen sind beispielsweise in den Schriften US 4803195, US 2006/0193812, WO 2005/055964, US 2004/0001891, EP 0384034 oder US 5508259 beschrieben. Diese Formulierungen weisen jedoch den Nachteil auf, dass es sich um 2-Phasensysteme handelt, die somit automatisch physikalisch instabil sind. Ferner ist die Herstellung entsprechend aufwendig, da der Geruchsstoff dispergiert und umhüllt werden muss. Auf der Haut liegt somit auch ein heterogenes System vor. Die verwendeten Emulgatoren wirken auch häufig auf der Haut irritierend.

Somit besteht die Notwendigkeit, Formulierungen zu entwickeln, die diese Nachteile nicht aufweisen. Es ist von entscheidender Bedeutung, dass die Zubereitung leicht auf die Haut aufgebracht werden kann und dort in relativ kurzer Zeit ein Depot ausbildet, das lange anhält, nicht oder wenig sichtbar ist, von Wasser nicht leicht abgewaschen wird und eine gewisse Schweißbeständigkeit hat, aber durch Seifenlösung leicht wieder entfernt werden kann.

Die aufgebrachten Depotformulierungen müssen zudem eine gewisse Wasserdampfdurchlässigkeit aufweisen, damit sich unter ihnen kein Feuchtedepot ausbildet, was zu ihrer Ablösung führen kann. Es ist somit auch ein großes Haftvermögen auf der Haut erforderlich.

Wasserlösliche Polymere alleine sind für den genannten Zweck nicht geeignet, da sie durch die Schweißproduktion der Haut aufgelöst werden und aufgrund ihrer Hygroskopizität klebrig werden. Sie erzeugen somit ein unangenehmes Hautgefühl und können auch an der Kleidung kleben.

Erfindungsgemäß wurde dieses Problem gelöst durch die Einarbeitung von Polymeren mit speziellen Eigenschaften und der Entwicklung von speziellen Darreichungsformen und Applikationsformen.

Demgemäß wurden Formulierungen zur kontrollierten Freisetzung von Geruchs- und Geschmacksstoffen für topische Anwendungen gefunden, enthaltend als Matrixbildner als wasserunlösliches Polymer ein Polyvinylacetat mit einem mittleren Molekulargewicht Mw von 200000 bis 700000 Dalton und als wasserlösliches Polymer ein Homopolymer des N-Vinylpyrrolidons mit einem K-Wert von 30.

Wasserunlöslich bedeutet erfindungsgemäß, dass sich unter Standardbedingungen (20°C, 0,1013 MPa) nicht mehr als 10 g in 1I Wasser lösen. Homopolymeren des N-Vinylpyrrolidons weisen einen K-Wert nach Fikentscher (gemessen in Wasser) von 30 auf.

Erfindungsgemäß werden für die Polymermatrix Mischungen von Polyvinylacetat und Polyvinylpyrrolidon mit einem K-Wert nach Fikentscher von 30 verwendet, d.h. Mischungen enthaltend als wasserunlösliches Polymer ein Polyvinylacetat mit einem mittleren Molekulargewicht Mw von 200.000 bis 700.000 Dalton und als wasserlösliches Polymer ein Homopolymer des N-Vinylpyrrolidons mit einem K-Wert von 30.

Gemäß einer bevorzugen Ausführungsform enthalten die Mischungen aus Polyvinylacetat und Polyvinylpyrrolidon zusätzlich 0.1 bis 2 Gew.-%, bezogen auf den Feststoffgehalt der Formulierung, an Natriumdodecylsulfat. Gemäß einer weiteren besonders bevorzugten Ausführungsform enthalten die Mischungen aus Polyvinylacetat und Polyvinylpyrolidon zusätzlich 0.1 bis 2 Gew.-%, bezogen auf den Feststoffgehalt der Formulierung, an Natriumdodecylsulfat und zusätzlich 0.05 bis 2 Gew.-%, bezogen auf den Feststoffgehalt der Formulierung, an Siliciumdioxid.

Die erfindungsgemäß verwendeten Matrix-Mischungen von wasserunlöslichen Polymeren wie Polyvinylacetat und wasserlöslichen Polymeren wie Polyvinylpyrrolidon können auf verschiedene Weise erhalten werden. Die Mischungen können sowohl als vorformuliertes Pulver eingesetzt werden oder direkt in einsatzfähiger Lösung hergestellt werden.

So können die wasserlöslichen Polymere und optional weitere Zusatzstoffe zu einen wässrigen Dispersion des wasserunlöslichen Polymeren gegeben werden. Weiterhin kann von vorneherein eine wässrige Dispersion der wasserunlöslichen Polymere unter Verwendung der wasser-löslichen Polymere als Schutzkolloid hergestellt werden. Oberflächenaktive Verbindungen wie Natriumdodecylsulfat können ebenfalls der wässrigen Dispersion des wasserunlöslichen Polymeren zugefügt werden Zu einer solchen wässrigen Dispersion kann dann eine zusätzliche Menge eines wasserlöslichen Polymers zugefügt werden.

Die erfindungsgemäß verwendeten wässrigen Dispersionen, enthaltend die wasserunlöslichen und wasserlöslichen Polymere, können durch übliche Sprühprozesse auch in Pulverform überführt werden.

Gemäß einer anderen Ausführungsform können die Mischungen aus wasserunlöslichen und wasserlöslichen Polymere durch gemeinsames Auflösen der jeweils pulverförmigen Polymere in einem geeigneten organischen Lösemittel oder organischwässrigen Lösemittelgemisch erhalten werden. Anschliessend kann eine Entfernung des Lösemittels durch geeignete Prozesse wie Sprühprozessse oder Verdampfungsprozessedurchgeführt werden, um wiederum eine pulverförmige Matrixmischung zu erhalten.

Besonders geeignete Mischungen sind kommerziell erhältlich, beispielsweise als Kollicoat ®SR30D oder Kollidon® SR.

Weiterhin können verschiedene andere Hilfsstoffe eingesetzt werden, um die Eigenschaften der Darreichungsform weiter zu verändern und zu verbessern. So können Farbstoffe hinzugefügt werden, wobei besonders bevorzugt Farbstoffe sind, die die Farbe der Haut erzeugen.

Als zusätzliche Hilfsstoffe können verwendet werden: Stabilisatoren, Konservierungsmittel, Weichmacher, Emulgatoren, Solubilisatoren, oberflächenaktive Stoffe, Spreitungsmittel, Stoffe zur Viskositätseinstellung, Stoffe zur Verbesserung der Zerstäubung.

Es können Geruchsstoffe unterschiedlicher Art und Anzahl eingearbeitet werden von einzelnen synthetischen Geruchsstoffen wie z.B. Geraniol, Linalool, Linalylacetat, Pyranol, Geranylacetat, Anisaldehyd, Citral, Citronellal, Lysmeral, Citronellol, Rosenoxid, Tetrahydrolinalool, Hydroxycitronellal, Betaionon oder Menthol bis hin zu komplexen Parfümkompositionen oder Insektenrepellents. Bevorzugt sind Geraniol, Linalool, Linalylacetat, Pyranol, Geranylacetat, Anisaldehyd, Citral, Citronellal, Lysmeral, Citronellol, Rosenoxid, Tetrahydrolinalool, Hydroxycitronellal, Betaionon oder Menthol. Besonders bevorzugt sind Geraniol, Linalool oder Menthol.

Die erfindungsgemäßen Formulierungen liegen in der Regel als Lösungen in verträglichen organischen Lösungsmitteln vor. Solche Lösungsmittel sind z.B. Ethanol, Propanol, Isopropanol, Ester wie Ethylacetat, Dimethylether, Pentan, Propan, Butan, Isobutan, halogenierte Treibgase oder Mischungen dieser Stoffe. Hierbei ist der Wassergehalt der Zubereitungen kleiner als 30%, vorzugsweise kleiner als 20% und besonders bevorzugt kleiner als 10%. Da es sich um Einphasensysteme handelt, sind die erfindungsgemäßen Zubereitungen physikalisch extrem stabil.

Eine weitere Ausführungsform der Erfindung bezieht sich auf Geruchsstoff-Formulierungen. Solche Formulierungen enthalten (i) 0,5 bis 10 Gew.-% Matrixbildner, (ii) 60 bis 99,4 Gew.-% Lösemittel, und (iii)0,1 bis 20 Gew.-% eines oder mehrerer Geruchsstoffe, wobei sich die Mengenangaben auf das Gesamtgewicht (100 Gew.-%) der Formulierung beziehen. Bevorzugte Formulierungen enthalten (i) 1,0 bis 5 Gew.-% Matrixbildner, (ii) 70 bis 98,5 Gew.-% Lösemittel, und (iii)0,5 bis 10 Gew.-% eines oder mehrerer Geruchsstoffe.

Die erfindungsgemäßen Zubereitungen können auf die Haut mittels verschiedener Applikatoren aufgebracht werden oder auch aufgesprüht werden. Durch den Anteil an organischem Lösungsmittel, das schnell verdampft, bildet sich in kurzer Zeit ein dünner Film aus, der als Depot fungiert. Die applikationsfertigen Zubereitungen können somit als Pumpspray, Aerosolspray, Tropflösung, Lösung angeboten werden. Der auf der Haut oder den Haaren entstehende Film ist klar, transparent, nicht klebrig, nicht oder kaum sichtbar und extrem flexibel, erzeugt kein Spannungsgefühl der Haut bei Bewegungen und gibt den Geruchstoff verzögert frei.

Bei besonders hoher Beladung mit Geruchsstoffen oder der Verwendung von unlöslichen Hilfsstoffen kann die flüssige Zubereitung auch aus einem zweiphasigen System bestehen, folglich einer Emulsion oder Dispersion, die dann durch Emulgatoren und Dispergatoren stabilisiert wird. Die Emulsionströpfchen bzw. festen Teilchen sind im Film auf der Haut feinst verteilt im Polymer eingebettet.

Die beschriebene Technologie kann auch eingesetzt werden, um Repellents gegen Insekten auf die menschliche oder tierische Haut zu applizieren. Dadurch wird ein verlängerter Schutz erreicht.

Die erfindungsgemäßen Zubereitungen ermöglichen auch die Behandlung von Textilien, wobei sich auch hier ähnliche Effekte einstellen wie z.B. keine Sichtbarkeit, retardierte Freisetzung der Geruchsstoffe und leichte Abwaschbarkeit in der Waschmaschine.

Überraschenderweise sind Polyvinylester mit unterschiedlichen Geruchsstoffen sehr gut verträglich und zwar sowohl in alkoholischer Lösung als auch in Form eines Filmes.

Die Eigenschaften der Filme können durch Kombination der wasserunlöslichen Polymeren mit wasserlöslichen Polymeren eingestellt werden, wobei eine hohe Verträglichkeit der Polymersorten vorhanden sein muss, da sich sonst keine transparenten Filme sondern opake Filme ausbilden.

Überraschenderweise weisen die Geruchstoffe eine hohe Affinität zu den Polymeren auf und dadurch werden diese verzögert über einen längeren Zeitraum freigesetzt.

### Beispiele

### Kollidon® SR:

80 % Polyvinylacetat (MW 450.000 D), 19 % Povidon K30, 0.8 % Natriumlaurylsulfat und 0.2 % Silica
Kollicoat® SR30D: 30 gew.-%ige wässrige Dispersion (in den Beispielen als Dispersion eingesetzt)
90 % Polyvinylacetat (MW 450.000 D), 9 % Povidon K30, 1 % Natriumlaurylsulfat, bez. auf Feststoff

Alle Prozentangaben sind Gew.-%.
Beispiele 1 - 4: Aerosolspray
Allgemeine Vorschrift
Das Menthol wurde vorab in Ethanol abs. aufgelöst. Danach wurde Kollicoat SR 30 D unter ständigem Rühren in die Ethanol abs./Mentholmischung zugegeben. Diese Zubereitung wurde in eine Aerosoldose gefüllt, die Aerosoldose mit Hilfe einer Bördelpresse verschlossen und durch das Ventil unter Druck Dimethylether zugegeben. Die Füllmenge betrug 100 g.

| Inhaltsstoffe | Beispiel 1 | Beispiel 2 |
|---|---|---|
| Kollicoat SR 30D | 13.33 % | 13.33 % |
| Ethanol abs | 36.67 % | 36.67 % |
| Dimethylether | 49.00 % | 48.00 % |
| Menthol | 1.00 % | 2.00 % |
| Total | 100.00 % | 100.00 % |

### Beispiels 3 - 4: Pumpsprays mit Ethylacetat

### Allgemeine Vorschrift

Das Menthol wurde vorab in Ethanol abs. aufgelöst. Danach wurde Kollicoat SR 30 D unter ständigem Rühren in die Ethanol abs./Mentholmischung zugegeben. Anschließend wurde Ethylacetat zugegeben und die resultierende Lösung für einige Minuten gerührt. Diese Zubereitung wurde in Pumpsprayflaschen aus Kunststoff abgefüllt. Die Füllmenge betrug 100 g.

| Inhaltsstoffe | Beispiel 3 | Beispiel 4 |
|---|---|---|
| Kollicoat SR 30D | 13.33 % | 13.33 % |
| Ethanol abs | 36.67 % | 36.67 % |
| Ethylacetat | 49.00 % | 48.00 % |
| Menthol | 1.00 % | 2.00 % |
| Total | 100.00 % | 100.00 % |

Alle hergestellten Pump- und Aerosolsprays (Beispiele 1-4) wurden auf Filterpapier und auf PE-Folie aufgesprüht. Nach kurzer Antrocknungszeit wurde eine sensorische Prüfung durchgeführt, ebenso nach 2 h und nach 24 h und die Ergebnisse dokumentiert.

### Aerosolspray

### Allgemeine Vorschrift

Der Aromastoff wurde vorab im Ethanol/Wasser Gemisch (9:1 V/V) aufgelöst. Danach wurde Kollidon SR unter ständigem Rühren in die Ethanol/Wassermischung zugegeben. Diese Zubereitung wurde in eine Aerosoldose gefüllt, die Aerosoldose verbördelt und durch das Ventil unter Druck Dimethylether zugegeben. Die Füllmenge betrug 50g.

Die sensorischen Eigenschaften wurden wie oben beschrieben durch Aufsprühen der Sprays auf einen Träger und nach den in den Tabellen angegebenen Zeiträumen überprüft.

### Geraniol - haltige Sprays und deren sensorische Eigenschaften

### Beispiel 5 - 7 Aerosolspray mit Geraniol:

| Inhaltsstoffe | Beispiel 5 | Beispiel 6 | Beispiel 7 |
|---|---|---|---|
| Kollidon SR | 2.00 % | 3.00 % | 5.00 % |
| Ethanol/Wasser (9:1) | 58.00 % | 57.00 % | 55.00 % |
| Dimethylether | 35.00 % | 35.00 % | 35.00 % |
| Geraniol | 5.00% | 5.00% | 5.00% |
| Total | 100.00 % | 100.00 % | 100.00 % |

### Aerosolspray mit Geraniol

| Formulierung | Anfang | 7h | 1 Tag | 5 Tage | 6-7 Tage | 8-11 Tage | 12-14 Tage |
|---|---|---|---|---|---|---|---|
| Geraniol Extra (5%ig in EtOH) | ✔ | ✔ | X | X | X | X | X |
| 2% Koll.SR/5 % Geraniol | ✔ | ✔ | ✔ | ✔ | ✔ | ✔ | ✔ |
| 3% Koll.SR/5 % Geraniol | ✔ | ✔ | ✔ | ✔ | ✔ | ✔ | ✔ |
| 5% Koll.SR/5 % Geraniol | ✔ | ✔ | ✔ | ✔ | ✔ | ✔ | ✔ |

| | | | | | | | |
|---|---|---|---|---|---|---|---|
| ✔ Geruch wahrnehmbar X Geruch nicht mehr wahrnehmbar | | | | | | | |

### Menthol - haltige Sprays und deren sensorische Eigenschaften

### Beispiels 8 - 11: Aerosolspray mit Menthol

| Inhaltsstoffe | Beispiel 8 | Beispiel 9 | Beispiel 10 | Beispiel 11 |
|---|---|---|---|---|
| Kollidon SR | 1.00 % | 2.00 % | 3.00 % | 5.00 % |
| Ethanol/Wasser (9:1) | 59.00 % | 58.00 % | 57.00 % | 55.00 % |
| Dimethylether | 39.00 % | 39.00 % | 39.00 % | 39.00 % |
| Menthol | 1.00% | 1.00% | 1.00% | 1.00% |
| Total | 100.00 % | 100.00 % | 100.00 % | 100.00 % |

### Aerosolspray mit Menthol

| Formulierung | Anfang | 1h | 4h | 7h | 1 Tag | 5 Tage | 6-7 Tage | 9-12 Tage | 13-14 Tage |
|---|---|---|---|---|---|---|---|---|---|
| Menthol (5%ig in E-tOH) | ✔ | ✔ | X | X | X | Nach 24h abgebrochen, rückstandsfreie Abtrocknung | | | |
| 1% Koll.SR/1 % Menthol | ✔ | ✔ | ✔ | ✔ | ✔ | ✔ | ✔ | X | X |
| 2% Koll.SR/1 % Menthol | ✔ | ✔ | ✔ | ✔ | ✔ | ✔ | ✔ | ✔ | X |
| 3% Koll.SR/1 % Menthol | ✔ | ✔ | ✔ | ✔ | ✔ | ✔ | ✔ | ✔ | ✔ |
| 5% Koll.SR/1 % Menthol | ✔ | ✔ | ✔ | ✔ | ✔ | ✔ | ✔ | ✔ | ✔ |

| | | | | | | | | | |
|---|---|---|---|---|---|---|---|---|---|
| ✔ Geruch wahrnehmbar ✔ X Geruch nicht mehr wahrnehmbar | | | | | | | | | |

### Mentholparfüm - haltige Sprays und deren sensorische Eigenschaften

### Beispiele 12 - 13: Aerosolspray mit Mentholparfüm

| Inhaltsstoffe | Beispiel 12 | Beispiel 13 |
|---|---|---|
| Kollidon SR | 2.00 % | 3.00 % |
| Ethanol/Wasser (9:1) | 58.00 % | 57.00 % |
| Dimethylether | 35.00 % | 35.00 % |
| Mentholparfüm | 5.00% | 5.00% |
| Total | 100.00 % | 100.00 % |

### Aerosolspray mit Mentholparfüm

| Formulierung | Anfang | 7h | 1 Tag | 5 Tage | 6-7 Tage | 8-11 Tage | 12-14 Tage |
|---|---|---|---|---|---|---|---|
| 2% Koll.SR/5 % Mentholparfüm | ✔ | ✔ | ✔ | ✔ | ✔ | ✔ | ✔ |
| 3% Koll.SR/5 % Mentholparfüm | ✔ | ✔ | ✔ | ✔ | ✔ | ✔ | ✔ |

| | | | | | | | |
|---|---|---|---|---|---|---|---|
| ✔ Geruch wahrnehmbar X Geruch nicht mehr wahrnehmbar | | | | | | | |

### Pumpsprays ohne Ethylacetat

### Allgemeine Vorschrift

Der Aromastoff wurde vorab im Ethanol/Wasser Gemisch (9:1 V/V) aufgelöst. Danach wurde Kollidon SR unter ständigem Rühren in die Ethanol/Wassermischung zugegeben.

### Geraniol - haltige Sprays und deren sensorische Eigenschaften

### Beispiels 14 - 16: Pumpspray mit Geraniol

| Inhaltsstoffe | Beispiel 14 | Beispiel 15 | Beispiel 16 |
|---|---|---|---|
| Kollidon SR | 2.00 % | 3.00 % | 5.00 % |
| Ethanol/Wasser (9:1) | 93.00 % | 92.00 % | 90.00 % |
| Geraniol | 5.00 % | 5.00 % | 5.00 % |
| Total | 100.00 % | 100.00 % | 100.00 % |

### Pumpspray mit Geraniol

| Formulierung | Anfang | 7h | 1 Tag | 5 Tage | 6-7 Tage | 8-11 Tage | 12-14 Tage |
|---|---|---|---|---|---|---|---|
| Geraniol (5%ig in E-tOH) | ✔ | ✔ | X | X | X | X | X |
| 2% Koll.SR/5 % Geraniol | ✔ | ✔ | ✔ | ✔ | ✔ | ✔ | X |
| 3% Koll.SR/5 % Geraniol | ✔ | ✔ | ✔ | ✔ | ✔ | ✔ | ✔ |
| 5% Koll.SR/5 % Geraniol | ✔ | ✔ | ✔ | ✔ | ✔ | ✔ | ✔ |

| | | | | | | | |
|---|---|---|---|---|---|---|---|
| ✔ Geruch wahrnehmbar X Geruch nicht mehr wahrnehmbar | | | | | | | |

### Mentholparfüm - haltige Sprays und deren sensorische Eigenschaften

### Beispiel 17 - 18 Pumpspray mit Mentholparfüm

| Inhaltsstoffe | Beispiel 17 | Beispiel 18 |
|---|---|---|
| Kollidon SR | 2.00 % | 3.00 % |
| Ethanol/Wasser (9:1) | 93.00 % | 92.00 % |
| Mentholparfüm | 5.00 % | 5.00 % |
| Total | 100.00 % | 100.00 % |

### Pumpspray mit Mentholparfüm

| Formulierung | Anfang | 7h | 1 Tag | 5 Tage | 6-7 Tage | 8-11 Tage | 12-14 Tage |
|---|---|---|---|---|---|---|---|
| 2% Koll.SR/5 % Mentholparfüm | ✔ | ✔ | ✔ | ✔ | ✔ | ✔ | ✔ |
| 3% Koll.SR/5 % Mentholparfüm | ✔ | ✔ | ✔ | ✔ | ✔ | ✔ | ✔ |

| | | | | | | | |
|---|---|---|---|---|---|---|---|
| ✔ Geruch wahrnehmbar X Geruch nicht mehr wahrnehmbar | | | | | | | |

Alle hergestellten Pump- und Aerosolsprays (Beispiele 5 - 18) wurden auf Glasplatten aufgesprüht. Nach kurzer Antrocknungszeit wurde eine sensorische Prüfung durchgeführt, ebenso nach 7 h, 1Tag, 5 Tagen, 6-7Tagen, 8-11Tagen und 12 -14 Tagen. Die Lagerung der Filme erfolgte für zwei Tage bei 33°C im Trockenschrank und anschließend bei einer Umgebungstemperatur von 23°C.

## Patentansprüche

1. Geruchs- und Geschmackstoff-Formulierungen für topische Anwendungen, enthaltend als Matrixbildner als wasserunlösliches Polymer ein Polyvinylacetat mit einem mittleren Molekulargewicht M_{w} von 200000 bis 700000 Dalton und als wasserlösliches Polymer ein Homopolymer des N-Vinylpyrrolidons mit einem K-Wert von 30.

2. Formulierung nach Anspruch 1, enthaltend als wasserunlösliches Polymer ein Polyvinylacetat mit einem mittleren Molekulargewicht M_{w} von 400.000 bis 500.000 Dalton und als wasserlösliches Polymer ein Homopolymer des N-Vinylpyrrolidons mit einem K-Wert von 30.

3. Formulierung nach Anspruch 1 oder 2, enthaltend zusätzlich 0.1 bis 2 Gew.-%, bezogen auf den Feststoffgehalt der Formulierung, an Natriumdodecylsulfat.

4. Formulierung nach einem der Ansprüche 1 bis 3, enthaltend zusätzlich 0.05 bis 2 Gew.-%, bezogen auf den Feststoffgehalt der Formulierung, an Siliciumdioxid.

5. Formulierung nach einem der Ansprüche 1 bis 4, enthaltend (i) 0,5 bis 10 Gew.-% Matrixbildner, (ii) 60 bis 99,4 Gew.-% Lösemittel, und (iii) 0,1 bis 20 Gew.-% eines oder mehrerer Geruchsstoffe, wobei sich die Mengenangaben auf das Gesamtgewicht der Formulierung beziehen.

6. Formulierung nach einem der Ansprüche 1 bis 4, enthaltend einen synthetischen Geruchsstoff aus der Gruppe bestehend aus Geraniol, Linalool, Linalylacetat, Pyranol, Geranylacetat, Anisaldehyd, Citral, Citronellal, Lysmeral, Citronellol, Rosenoxid, Tetrahydrolinalool, Hydroxycitronellal, Betaionon und Menthol.

7. Verwendung einer Formulierung gemäß einem der Ansprüche 1 bis 5 in Sprays für topische Anwendungen.

8. Verwendung einer Formulierung gemäß einem der Ansprüche 1 bis 5 als Dermalspray für pharmazeutische oder kosmetische Anwendungen an Mensch und Tier.

9. Verwendung einer Formulierung gemäß einem der Ansprüche 1 bis 5 zur Textilbehandlung.

## Claims

1. An odorant and flavoring formulation for topical application comprising, as matrix former, a polyvinyl acetate having an average molecular weight M_{w} of 200 000 to 700 000 daltons as water-insoluble polymer and a homopolymer of N-vinylpyrrolidone having a K value of 30 as water-soluble polymer.

2. The formulation according to claim 1, comprising a polyvinyl acetate having an average molecular weight M_{w} of 400 000 to 500 000 daltons as water-insoluble polymer and a homopolymer of N-vinylpyrrolidone having a K value of 30 as water-soluble polymer.

3. The formulation according to claim 1 or 2, additionally comprising 0.1 to 2 wt% sodium dodecylsulfate, based on the solids content of the formulation.

4. The formulation according to any of claims 1 to 3, additionally comprising 0.05 to 2 wt% silicon dioxide, based on the solids content of the formulation.

5. The formulation according to any of claims 1 to 4, comprising (i) 0.5 to 10 wt% matrix formers, (ii) 60 to 99.4 wt% solvent, and (iii) 0.1 to 20 wt% of one or more odorants, wherein the amounts are based on the total weight of the formulation.

6. The formulation according to any of claims 1 to 4, comprising a synthetic odorant from the group consisting of geraniol, linalool, linalyl acetate, pyranol, geranyl acetate, anisaldehyde, citral, citronellal, lysmeral, citronellol, rose oxide, tetrahydrolinalool, hydroxycitronellal, beta-ionone and menthol.

7. The use of a formulation according to any of claims 1 to 5 in sprays for topical applications.

8. The use of a formulation according to any of claims 1 to 5 as a dermal spray for pharmaceutical or cosmetic applications to humans and animals.

9. The use of a formulation according to any of claims 1 to 5 for textile treatment.

## Revendications

1. Formulations de substance odorante et aromatisante pour des applications topiques, contenant, en tant qu'agent formant une matrice, un poly(acétate de vinyle) doté d'un poids moléculaire moyen M_{w} de 200 000 à 700 000 daltons en tant que polymère insoluble dans l'eau et un homopolymère de la N-vinylpyrrolidone doté d'une valeur K de 30 en tant que polymère soluble dans l'eau.

2. Formulation selon la revendication 1, contenant un poly(acétate de vinyle) doté d'un poids moléculaire moyen M_{w} de 400 000 à 500 000 daltons en tant que polymère insoluble dans l'eau et un homopolymère de la N-vinylpyrrolidone doté d'une valeur K de 30 en tant que polymère soluble dans l'eau.

3. Formulation selon la revendication 1 ou 2, contenant de plus 0,1 à 2 % en poids, par rapport à la teneur en solides de la formulation, de dodécylsulfate de sodium.

4. Formulation selon l'une quelconque des revendications 1 à 3, contenant de plus 0,05 à 2 % en poids, par rapport à la teneur en solides de la formulation, de dioxyde de silicium.

5. Formulation selon l'une quelconque des revendications 1 à 4, contenant (i) 0,5 à 10 % en poids d'agent formant une matrice, (ii) 60 à 99,4 % en poids de solvant, et (iii) 0,1 à 20 % en poids d'une ou plusieurs substances odorantes, les données quantitatives se rapportant au poids total de la formulation.

6. Formulation selon l'une quelconque des revendications 1 à 4, contenant une substance odorante synthétique du groupe constitué par le géraniol, le linalool, l'acétate de linalyle, le pyranol, l'acétate de géranyle, l'anisaldéhyde, le citral, le citronellal, le lysméral, le citronellol, l'oxyde de rose, le tétrahydrolinalool, l'hydroxycintronellal, la bêtaionone et le menthol.

7. Utilisation d'une formulation selon l'une quelconque des revendications 1 à 5 dans des sprays pour des applications topiques.

8. Utilisation d'une formulation selon l'une quelconque des revendications 1 à 5 en tant que spray dermique pour des applications pharmaceutiques ou cosmétiques sur l'homme et l'animal.

9. Utilisation d'une formulation selon l'une quelconque des revendications 1 à 5 pour le traitement de textiles.
